(19) Europäisches Patentamt
European Patent Office
Office européen des brevets

(11) Veröffentlichungsnummer: **0 137 329**
**B1**

# (12) EUROPÄISCHE PATENTSCHRIFT

(45) Veröffentlichungstag der Patentschrift: **22.04.87**

(21) Anmeldenummer: **84110834.3**

(22) Anmeldetag: **11.09.84**

(51) Int. Cl.⁴: **C 07 C 149/36,** C 08 G 63/68, C 08 G 63/76, C 09 K 15/14, C 08 K 5/37 // C08L25/06

(54) **Polymere 4-Hydroxyphenylthioverbindungen.**

(30) Priorität: **14.09.83 US 531876**

(43) Veröffentlichungstag der Anmeldung: **17.04.85 Patentblatt 85/16**

(45) Bekanntmachung des Hinweises auf die Patenterteilung: **22.04.87 Patentblatt 87/17**

(84) Benannte Vertragsstaaten: **DE FR GB IT**

(56) Entgegenhaltungen: **EP - A - 0 079 855**

(73) Patentinhaber: **CIBA-GEIGY AG, Klybeckstrasse 141, CH-4002 Basel (CH)**

(72) Erfinder: **Spivack, John D., 1 Blue Jay Street, Spring Valley New York 10977 (US)**
Erfinder: **Pastor, Stephen D., 112 Union Road, Spring Valley New York 10977 (US)**

(74) Vertreter: **Zumstein, Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Anmeldung betrifft neue polymere 4-Hydroxyphenylthioverbindungen, deren Verwendung als Stabilisatoren für organisches Material sowie die damit stabilisierten Zusammensetzungen.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrates oder des Stabilisators ablaufen, die zu neuen Chromophoren führen. Weiterhin müssen Probleme der Verarbeitungsstabilität und der Substratverträglichkeit beachtet werden.

Überraschenderweise ist jetzt gefunden worden, dass die polymeren 4-Hydroxyphenylthioverbindungen dieser Erfindung eine ungewöhnliche Kombination wünschenswerter Eigenschaften besitzen, wodurch diese Stoffe zu besonders effektiven Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Aus der EP-A-79 855 ist eine Klasse von monomeren Mercaptophenolderivaten bekannt. Es handelt sich dabei um alkylierte Hydroxyphenylthioalkansäureester, die sich zum Stabilisieren von organischem Material gegen lichtinduzierten und thermooxidativen Abbau eignen.

2-Hydroxyphenylthioreste enthaltende Polyester sind im US Patent 3 832 329 als Zwischenprodukte für die Herstellung von Polyurethanen, für den Einsatz als chemisch gebundene Weichmacher in Polymeren oder als Vernetzungsprodukte in Polycarbonaten beschrieben.

Die vorliegende Erfindung betrifft Verbindungen der Formel I

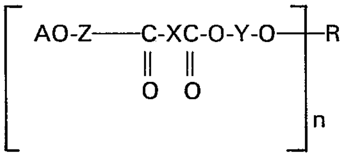

(I),

worin A $C_1$-$C_{12}$ Alkyl, $C_5$-$C_6$ Cycloalkyl, unsubstituiertes oder durch $C_1$-$C_{12}$ Alkyl substituiertes Phenyl oder eine Gruppe

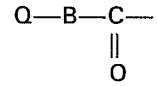

bedeutet, worin Q eine Gruppe der Formel

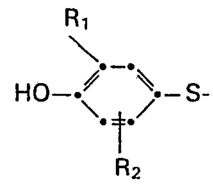

ist und B $C_2$-$C_6$ Alkylen bedeutet,

$R_1$ und $R_2$ unabhängig voneinander Wasserstoff, $C_1$-$C_{18}$ Alkyl, $C_5$-$C_6$ Cycloalkyl, Phenyl, durch $C_1$-$C_{12}$ Alkyl substituiertes Phenyl, $C_7$-$C_9$ Aralkyl oder durch $C_1$-$C_{12}$ Alkyl substituiertes $C_7$-$C_9$ Aralkyl sind,

X als gegebenenfalls wiederholt vorkommendes Symbol jeweils gleich oder verschieden $C_2$-$C_{10}$ Alkylen, durch eine Gruppe Q substituiertes $C_2$-$C_{10}$ Alkylen, $C_6$-$C_{10}$ Arylen bedeutet,

Y als gegebenenfalls wiederholt vorkommendes Symbol jeweils gleich oder verschieden ununterbrochenes oder durch ein oder zwei Sauerstoffatome unterbrochenes, unsubstituiertes oder durch eine Gruppe -O-A substituiertes $C_2$-$C_6$ Alkylen bedeutet,

Z eine direkte Bindung oder $C_2$-$C_6$ Alkylenoxy ist, wobei das Sauerstoffatom an die Carbonylgruppe gebunden und der Alkylenrest ununterbrochen oder durch ein oder zwei Sauerstoffatome unterbrochen ist,

R Wasserstoff oder eine Gruppe A bedeutet,

n eine Zahl von 1 bis 100 ist,

mit der Bedingung, dass mindestens eines von X und Y eine Gruppe Q enthält.

$C_1$-$C_{12}$ Alkylreste bedeuten geradkettiges oder verzweigtes Alkyl, wie z.B. Methyl, Ethyl, n-Propyl, Isopropyl, n-Butyl, sek.-Butyl, tert.-Butyl, n-Pentyl, tert.-Pentyl, n-Hexyl, 2-Ethylhexyl, n-Octyl, 1,1,3,3-Tetramethylbutyl, Decyl oder Dodecyl. Bevorzugt sind $C_1$-$C_8$ Alkylreste. $R_1$ und $R_2$ als $C_1$-$C_{18}$ Alkyl bedeuten zusätzlich noch z.B. Tridecyl, Tetradecyl, Hexadecyl und Octadecyl.

$C_5$-$C_6$ Cycloalkyl bedeutet Cyclopentyl oder Cyclohexyl.

$C_2$-$C_6$ Alkylen ist z.B. Methylen, Ethylen, Propylen, Trimethylen, 2,2-Dimethylpropan-1,3-diyl, Tetramethylen, Pentamethylen oder Hexamethylen. Durch ein oder zwei Sauerstoffatome unterbrochenes Alkylen ist beispielsweise 3-Oxapentamethylen, 4-Oxaheptamethylen oder 3,6-Dioxa-octamethylen. X als $C_2$-$C_{10}$ Alkylen bedeutet zusätzlich auch z.B. Octamethylen oder Decamethylen.

Bedeuten $R_1$ und $R_2$ Aralkyl, so handelt es sich z.B. um Benzyl, α-Methylbenzyl oder α,α-Dimethylbenzyl.

X als $C_6$-$C_{10}$ Arylen ist z.B. ein von Phenyl, Tolyl, Mesityl, Xylyl und 1- oder 2-Naphthyl abgeleiteter Rest.

Von Interesse sind Verbindungen der Formel I, worin n die Zahl 1 bedeutet.

Bevorzugt werden die Verbindungen der Formel I, worin A $C_1$-$C_8$ Alkyl, Phenyl oder eine Gruppe der Formel

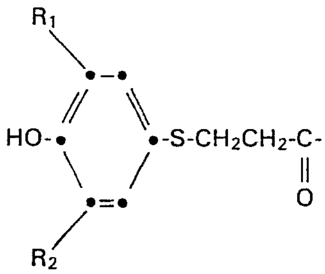

ist, $R_1$ und $R_2$ unabhängig voneinander $C_1$-$C_8$ Alkyl bedeuten,

X als gegebenenfalls wiederholt vorkommendes Symbol jeweils gleich oder verschieden $C_2$-$C_6$ Alkylen, welches durch eine Gruppe Q' der Formel

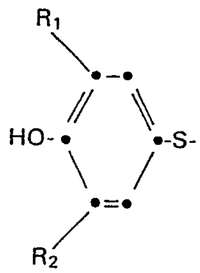

substituiert ist, worin $R_1$ und $R_2$ die in dieser Bevorzugung bereits angegebene Bedeutung haben, oder Phenylen bedeutet,

Y als gegebenenfalls wiederholt vorkommendes Symbol jeweils gleich oder verschieden unsubstituiertes oder durch eine Gruppe -O-A, worin A die in dieser Bevorzugung bereits angegebene Bedeutung hat, substituiertes $C_2$-$C_6$ Alkylen ist oder 3-Oxapentamethylen bedeutet,

Z eine direkte Bindung, $C_2$-$C_6$ Alkylenoxy oder 3-Oxapentamethylenoxy ist,

R Wasserstoff oder eine Gruppe A ist, wobei A die in dieser Bevorzugung bereits angegebene Bedeutung hat,

n die Zahlen 1 bis 50 ist,

mit der Bedingung dass,

wenn n = 1 ist, mindestens eines von X oder Y eine Gruppe Q' enthält und,

wenn n = 2-50 ist, mindestens zwei von A, X oder Y eine Gruppe Q' enthalten.

Besonders bevorzugt sind Verbindungen der Formel I, worin A $C_1$-$C_8$ Alkyl oder eine Gruppe der Formel

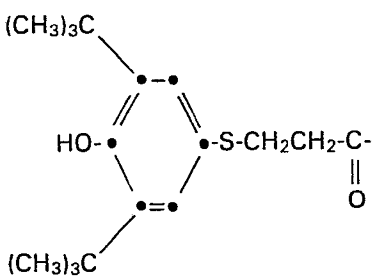

bedeutet,

X als gegebenenfalls wiederholt vorkommendes Symbol jeweils gleich oder verschieden $C_2$-$C_6$ Alkylen, 1,2-Phenylen oder eine Gruppe der Formel

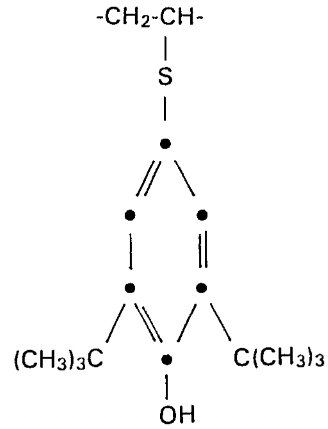

ist,

Y als gegebenenfalls wiederholt vorkommendes Symbol jeweils gleich oder verschieden $C_2$-$C_6$ Alkylen, 3-Oxapentamethylen oder eine Gruppe der Formel

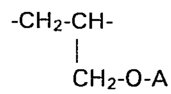

worin

A die in dieser Bevorzugung angegebene Bedeutung hat, bedeutet,

Z eine direkte Bindung, Ethylenoxy oder 3-Oxapentamethylenoxy ist,

R Wasserstoff oder A ist, wobei A die in dieser Bevorzugung bereits angegebene Bedeutung hat,

n die Zahlen 2 bis 25 bedeutet,

mit der Bedingung, dass mindestens zwei von A, X oder Y eine Gruppe der Formel

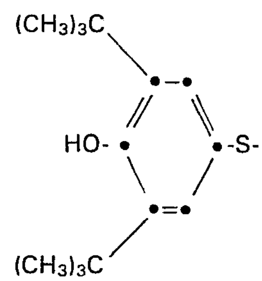

enthalten.

Die Polymeren der vorliegenden Erfindung können erhalten werden durch Addition von (II)

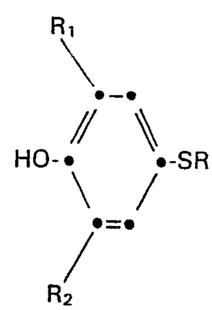

(II),

worin $R_1$ und $R_2$ die oben angegebene Bedeutung haben, zu Polymeren, die durch Umsetzung eines Alkohols mit einem Epoxyd und einem Anhydrid erhalten werden, wobei mindestens einer dieser drei Ausgangsprodukte einen α,β-ungesättigten Carbonylrest enthalten muss. Eine weitere Möglichkeit ist, dass (II) zuerst an den α,β-ungesättigten Rest addiert und dann erst polymerisiert wird. Dazu kann (II) zuerst zu einem der nachstehend aufgelisteten Monomeren addiert werden und nachträglich zu einer erfindungsgemässen Verbindung polymerisiert werden.

Geeignete gesättigte Alkohole sind primäre und sekundäre Alkohole mit 1 bis 12 C-Atomen, wie z.B. Methanol, Ethanol, Propanol, Butanol, Cyclohexanol sowie Phenole.

Geeignete ungesättigte Alkohole sind z.B. Acrylsäure-hydroxyethylester, Acrylsäurehydroxypropylester, Methacrylsäure-hydroxyethylester, Crotonsäure-hydroxyethylester.

Geeignete gesättigte Epoxide sind z.B. solche mit 2 bis 6 C-Atomen, insbesondere Ethylenoxid, Propylenoxid, Epichlorhydrin, Cyclohexenoxid, Butylglycidylether.

Geeignete ungesättigte Epoxide sind z.B. die Glycidylester von α,β-ungesättigten Carbonsäuren der Formel

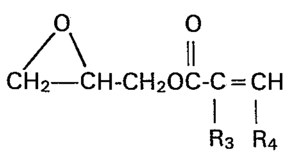

worin $R_3$ und $R_4$ unabhängig voneinander Methyl oder Wasserstoff bedeuten. Besonders geeignete Ester sind beispielsweise Acrylsäure-2,3-epoxypropyl, Methacrylsäure-2,3-epoxypropyl und Crotonsäure-2,3-epoxypropyl.

Anhydride, die zur Herstellung der erfindungsgemässen Polymeren geeignet sind, sind z.B. solche mit 4 bis 12 C-Atomen, insbesondere Bernsteinsäureanhydrid, Phthalsäureanhydrid und Maleinsäureanhydrid.

Polymere mit Carboxylendgruppen können mit den obengenannten α,β-ungesättigten Epoxiden umgesetzt und dann (II) addiert werden. Prepolymere mit Hydroxylendgruppen können zur Umesterung von z.B. Acryl- oder Methacrylsäureestern verwendet und anschliessend (II) addiert werden.

Eine besonders bevorzugte Ausführungsform ist die Umesterung einer Verbindung der Formel

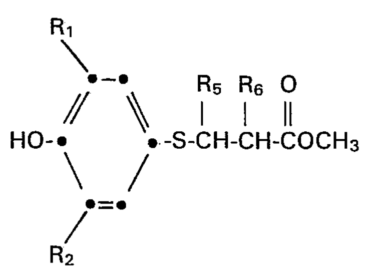

worin $R_1$ und $R_2$ die oben angegebene Bedeutung haben, $R_5$ Wasserstoff oder Methyl und $R_6$ Wasserstoff, Methyl oder Ethyl sind, mit reaktive Hydroxygruppen enthaltenden Prepolymeren.

Alle oben beschriebenen Reaktionen, die Kondensations-, Veresterungs- oder Umesterungsreaktionen sind, können in Analogie zu bekannten Methoden durchgeführt werden.

Die erfindungsgemässen Verbindungen lassen sich als effektive Stabilisatoren für Kunststoffe, Polymere oder Harze einsetzen. Sie lassen sich als Stabilisatoren in Mineralölen oder synthetischen Ölen verwenden.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I als Stabilisator, wobei als organisches Material vorzugsweise ein Polymer zu verstehen ist.

Vorzugsweise verwendet man die Verbindungen der Formel I als Stabilisatoren für Polyolefine wie beispielsweise Polyethylen oder Polypropylen; Polystyrol, vorzugsweise schlagfestes Polystyrol; ABS-Harz; Elastomere wie beispielsweise Polybutadien, EPM, EPDM, SBR, Nitril-Kautschuk oder natürlicher Kautschuk.

Polymere, die mit den erfindungsgemässen Verbindungen stabilisiert werden können, sind beispielsweise:

1. Polymere von Mono- und Diolefinen, beispielsweise Polyethylen (das gegebenenfalls vernetzt sein kann), Polypropylen, Polyisobutylen, Polybutylen-1, Polymethylpenten-1, Polyisopropen oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Polym-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyesteracrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrigen Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Stabilisatoren werden den Kunststoffen in Mengen von 0,01 bis 5 Gew.-% bezogen auf das stabilisierte Material zugemischt. Je nach Verwendung und Substrat wird die Stabilisatormenge variiert. Bevorzugt verwendet man 0,05 bis 2 Gew.-% des Stabilisators, besonders bevorzugt 0,1 bis 1 Gew.-%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine

Suspension des Stabilisators kann mit einer Lösung, Suspension oder Emulsion des Polymeren vermischt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z.B. weitere aminische Antioxidantien, Metallpassivatoren, Rostinhibitoren, Viskositäts-Index Verbesserer, Stockpunkterniedriger, Dispergiermittel/Tenside und Verschleissschutzadditive. Als weitere Additive, mit denen zusammen die erfindungsgemäss verwendeten Stabilisatoren eingesetzt werden können, sind beispielsweise zu nennen:

*1. Antioxidantien*

*1.1 Alkylierte Monophenole*

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

*1.2 Alkylierte Hydrochinone*

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

*1.3. Hydroxylierte Thiodiphenylether*

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

*1.4 Alkyliden-Bisphenole*

2,2'-Methylen-bis-(6-tert-butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylohenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methyliden-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

*1.5 Benzylverbindungen*

1,3,5-Tri-(3,5-di-tert.butyl-4-hydroxybenzyl)-2,4,6-trimethylbenzol
Di-(3,5-di tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)-dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

*1.6. Acylaminophenole*

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

*1.7. Ester der β-(3,5-Di-tert.butyl-4-hydroxyphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*1.8. Ester der β-(5-tert.butyl-4-hydroxy-3-methylphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*1.9. Amide der β-(3,5-Di-tert.butyl-4-hydroxyphenyl)-propionsäure,* wie z.B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin.

*2. UV-Absorber und Lichtschutzmittel*

*2.1. 2-(2'-Hydroxyphenyl)-benztriazole,* wie z.B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert-butyl-5'-methyl-, 3'-sec.-Butyl-5'-tert.butyl, 4'-Octoxy-, 3',5'-Di-ter.amyl-, 3',5'-Bis-(α,α-dimethylbenzyl)-Derivat.

*2.2. 2-Hydroxybenzophenone,* wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

*2.3. Ester von gegebenenfalls substituierten Benzoesäuren,* wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert-butylphenylester, 3,5-Di-tert.butyl-4-hydroxybenzoesäurehexadecylester.

*2.4. Acrylate,* wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methylindolin.

*2.5. Nickelverbindungen,* wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

*2.6. Sterisch gehinderte Amine,* wie z.B. Bis-(2,2,6,6-tetramethylpiperidyl)-sebacat, Bis-(1,2,2,-6,6-pentamethylpiperidyl)-sebacat, n-Butyl-3,5-di-tert.butyl-4-hydroxybenzyl-malonsäure-bis(1,2,2,-6,6-pentamethylpiperidyl)ester, Kondensationsprodukt aus 1-Hydroxyethyl-2,2,6,6-Tetramethyl-4--hydroxypiperidin und Bernsteinsäure, Kondensationsprodukt aus N,N'-(2,2,6,6-Tetramethyl-4-piperidyl)-hexamethylendiamin und 4-tert.Octylamino--2,6-dichlor-1,3,5-s-triazin, Tris-(2,2,6,6-tetramethyl-4-piperidyl)-nitrilotriacetat, Tetrakis-(2,2,6,6--tetramethyl-4-piperidyl)-1,2,3,4-butantetracarbonsäure, 1.1'-(1,2-Ethandiyl)-bis-(3,3,5,5-tetramethyl-piperazinon).

*2.7. Oxalsäurediamide,* wie z.B. 4,4'-octyloxy-oxanilid, 2,2'-Di-octyloxy-5,5'-d-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

*3. Metalldesaktivatoren,* wie z.B. N,N'-Diphenyloxalsäurediamid, N-Salicylal-N'-salicyloylhydrazin, N,N'-Bis-salicyloylhydrazin, N,N'-Bis-(3,5-di-tert.-butyl-4-hydroxyphenylpropionyl)-hydrazin, 3-Salicyloylamino-1,2,4-triazol, Bis-benzyliden-oxalsäuredihydrazid.

*4. Phosphate und Phosphonite,* wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di--(2,4-di-tert.butylphenyl)-pentaerythritdiphosphit, Tristearyl-sorbit-triphosphit, Tetrakis-(2,4-di-tert.-butylphenyl)-4,4'-biphenylen-diphosphonit.

*5. Peroxidzerstörende Verbindungen,* wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmer capto)-propionat.

*6. Polyamidstabilisatoren,* wie z.B. Kupfersalze in Kombination mit Jodiden und/oder Phosphorverbindungen und Salze des zweiwertigen Mangans.

*7. Basische Co-Stabilisatoren,* wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

*8. Nukleierungsmittel,* wie z.B. 4-tert.Butylbenzoesäure, Adipinsäure, Diphenylessigsäure.

*9. Füllstoffe und Verstärkungsmittel,* wie z.B. Calciumcarbonat, Silikate, Glasfasern, Asbest, Talk, Kaolin, Glimmer, Bariumsulfat, Metalloxide und -hydroxide, Russ, Graphit.

*10. Sonstige Zusätze,* wie z.B. Weichmacher, Gleitmittel, Emulgatoren, Pigmente, Optische Aufheller, Flammschutzmittel, Antistatika, Treibmittel.

Für den Einsatz zusammen mit den erfindungsgemässen Verbindungen bevorzugte Costabilisatoren sind Antioxidantien, Lichtschutzmittel, Metalldesaktivatoren, Phosphite, Phosphonite und Thiosynergisten (peroxidzerstörende Verbindungen).

Die folgenden Beispiele erläutern die Erfindung. Alle Mengenangaben entsprechen Gewichtsteilen soweit nicht anderweitig angegeben.

*Beispiel 1*

In einem mit einem Dewar-Kühler (Aceton-Trokkeneis) versehenen 500 ml-Kolben wird ein Gemisch aus 7,41 g n-Butylalkohol, 49,03 g Maleinsäureanhydrid, 29,04 g Propylenoxid, 0,69 g Tetrabutylammoniumchlorid und 0,49 g Paramethoxyphenol auf 75°C erhitzt und bei dieser Temperatur gehalten, bis eine Säurezahl von 1,0 mg KOH/g Polymer erreicht ist (ca. 13 Stunden). IR (1% in Chloroform): 1740 $cm^{-1}$ (Ester-Carbonyl), 1640 $cm^{-1}$ (Doppelbindung).

Man erhält eine Verbindung der Formel

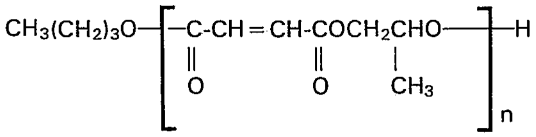

n = 5

*Beispiel 1a*

Beispiel 1 wird wiederholt, mit der Ausnahme, dass 9,8 g anstelle von 49,03 g Maleinsäureanhydrid und 5,8 anstelle von 29,04 g Propylenoxid eingesetzt werden. Man erhält eine Verbindung der Formel

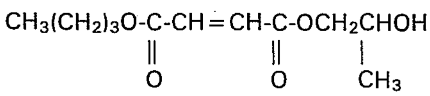

*Beispiel 2*

In einem 100 ml-Kolben wird unter Stickstoffzufuhr eine Lösung von 8,54 g des Polymeren aus Beispiel 1 in 2,5 ml Toluol mit einer Lösung von 11,93 g 2,6-Di-tert.-butyl-4-mercaptophenol in 25 ml Toluol und anschliessend mit 0,2 g Triethylamin behandelt. Das Lösungsmittel wird im Vakuum abdestilliert und das Produkt chromatographisch gereinigt. Das IR-Spektrum zeigt keine Doppelbindung bei 1640 $cm^{-1}$. Man erhält das Polymere der Formel

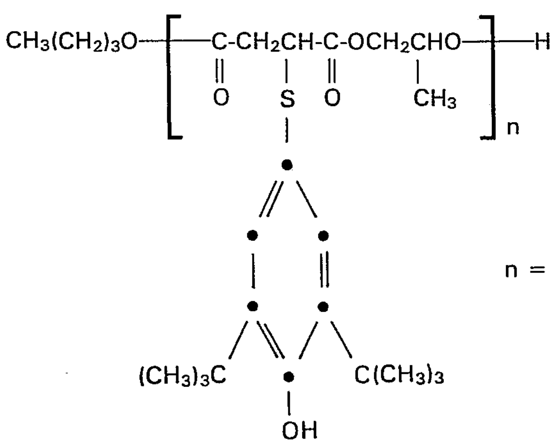

n = 5

Anal. Berechnet für $[C_{21}H_{30}O_5S]_n$: S, 7,8.
Gefunden: S, 7,5.

*Beispiel 2a*

Beispiel 2 wird wiederholt mit der Ausnahme, dass 2,3 g der Verbindung aus Beispiel 1a anstelle von 8,54 g der Verbindung aus Beispiel 1 und 2,38 g anstelle von 11,92 g 2,6-Di-tert.-butyl-4-mercaptophenol eingesetzt werden. Man erhält eine Verbindung der Formel

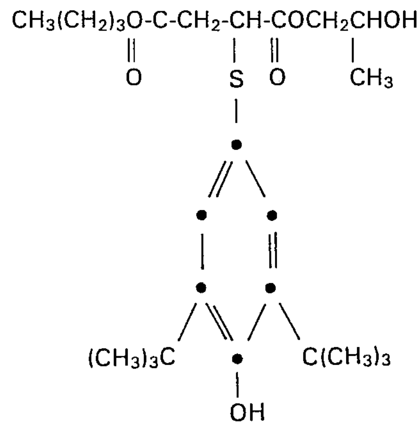

*Beispiel 3*

Das Verfahren von Beispiel 1 wird wiederholt, indem man 2,22 g n-Butylalkohol, 14,71 g Maleinsäureanhydrid, 19,3 g Butylglycidyl-ether, 0,2 g Tetrabutylammoniumchlorid und 0,04 g Paramethoxyphenol reagieren lässt, bis die Säurezahl 1,7 mg KOH/g erreicht ist. Man erhält ein Produkt der Formel

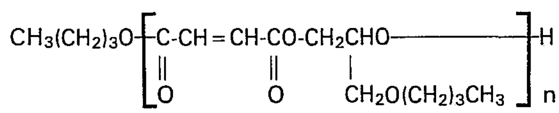

n = 5

*Beispiel 4*

Das Verfahren von Beispiel 2 wird wiederholt indem man 12,15 g der Verbindung aus Beispiel 3, 11,92 g 2,6-Di-tert.-butyl-4-mercaptophenol und 1,0 g Triethylamin reagieren lässt. Das Rohprodukt wird chromatographisch gereinigt. Das IR-Spektrum (1% in Chloroform) zeigt Ester-Carbonyl bei 1740 $cm^{-1}$ und keine Doppelbindung. Man erhält ein Produkt der Formel

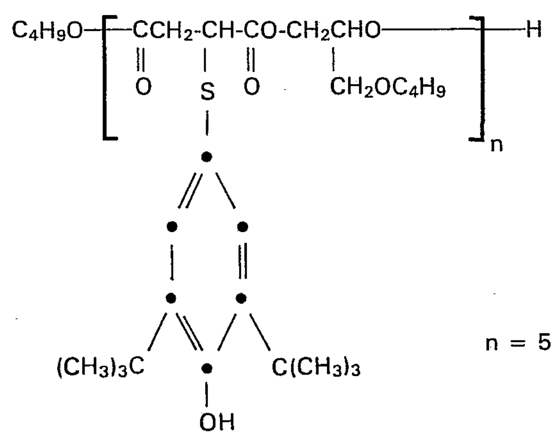

n = 5

Anal. Berechnet: S, 6,6.
Gefunden: S, 6,0.

*Beispiel 5*

Das Verfahren von Beispiel 1 wird wiederholt, indem man 11,61 g Acrylsäure-2-hydroxyethylester, 38,44 g Acrylsäure-glycidylester, 30,02 g Bernsteinsäureanhydrid, 0,4 g Benzyl-triethylammonium-chlorid und 0,08 g 2,6-Di-tert.-butyl-4-methylphenol reagieren lässt, bis die Säurezahl 1,4 mg KOH/g erreicht ist.

IR (1% in Chloroform): 1630 $cm^{-1}$ (Doppelbindung). Man erhält ein Produkt der Formel

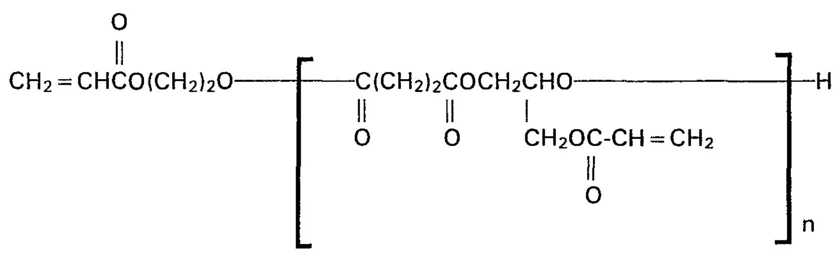

n = 3

*Beispiel 6*

Das Verfahren von Beispiel 2 wird wiederholt indem man 16,01 g der Verbindung aus Beispiel 5, 19,07 g 2,6-Di-tert.-butyl-4-mercaptophenol und 0,5 g Triethylamin reagieren lässt. Das Rohprodukt wird chromatographisch gereinigt.

Das IR-Spektrum zeigt keine Doppelbindung. Man erhält ein Produkt der Formel

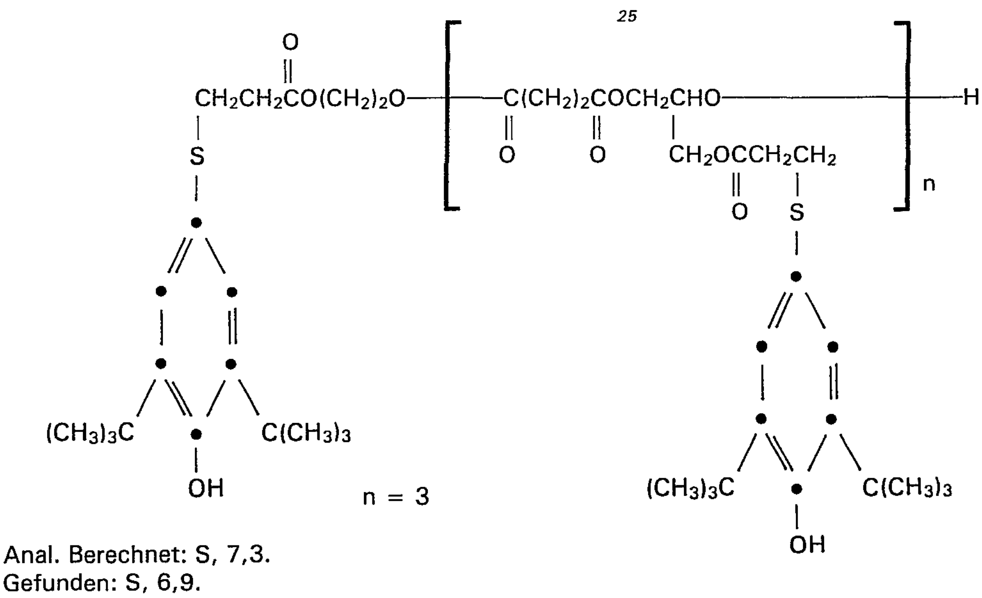

n = 3

Anal. Berechnet: S, 7,3.
Gefunden: S, 6,9.

*Beispiel 7*

Das Verfahren von Beispiel 1 wird wiederholt, indem man 11,6 g Acrylsäure-2-hydroxyethylester, 38,4 g Acrylsäure-glycidylester, 29,4 g Maleinsäureanhydrid, 0,4 g Benzyl-triethylammoniumchlorid und 0,08 g 2,6-Di-tert.-butyl-4-methylphenol reagieren lässt, bis die Säurezahl 0,9 mg KOH/g erreicht wird. IR (1% in Chloroform): 1735 $cm^{-1}$ (Carbonyl), 1640 $cm^{-1}$ (Doppelbindung). Man erhält die Verbindung der Formel

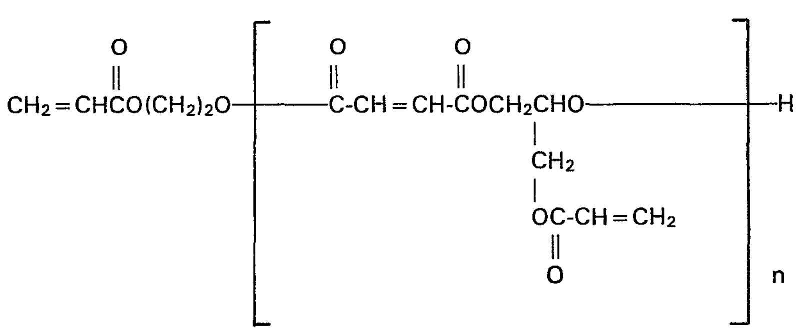

n = 3

*Beispiel 8*

Das Verfahren von Beispiel 2 wird wiederholt, indem man 7,9 g der Verbindung aus Beispiel 7, 16,7 g 2,6-Di-tert.-butyl-4-mercaptophenol und 0,5 g Triethylamin reagieren lässt. Das Rohprodukt wird chromatographisch gereinigt. Das IR-Spektrum zeigt keine Doppelbindung. Man erhält die Verbindung der Formel

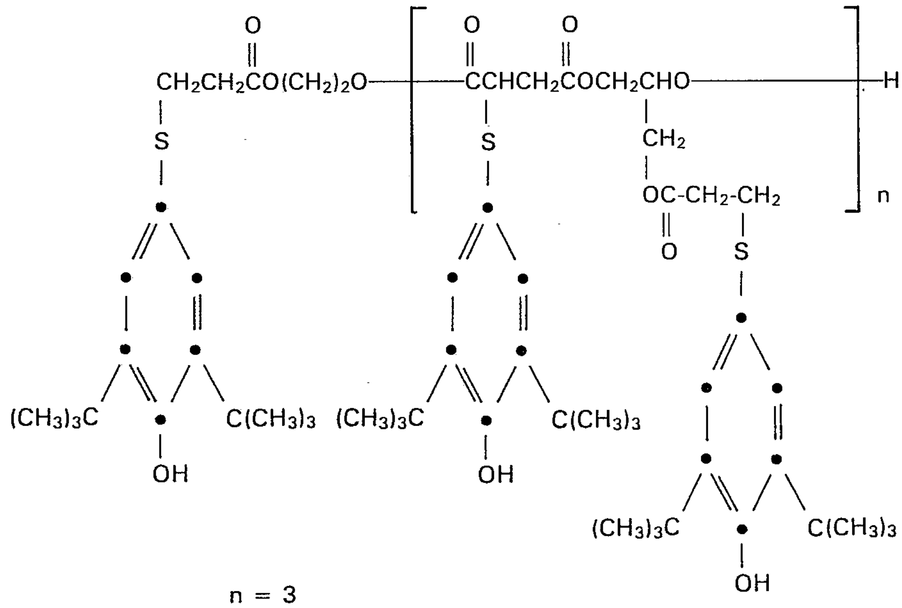

n = 3

Anal. Berechnet: S, 9,1.
Gefunden: S, 8,7.

*Beispiel 9*

Ein Gemisch aus 19,15 g 2-(3,5-Di-tert.-butyl-4--hydroxyphenylthio)-bernsteinsäure-dimethylester, 4,51 g 1,4-Butandiol und 0,24 g Tetrabutyltitanat wird unter Stickstoffzufuhr auf 130°C erhitzt und das sich bildende Methanol in einer Kühlfalle (Aceton-Trockeneis) abgefangen. Der Druck wird allmählich auf 0,13 mbar reduziert und die Temperatur auf 160°C erhöht. Das Produkt wird in 100 ml heissem Tetrahydrofuran gelöst, zu welchem 14 ml Wasser zugegeben werden. Man filtriert und destilliert das Lösungsmittel im Vakuum ab. Der Rückstand wird in Hexan zermahlen. Das erhaltene Polymere der Formel

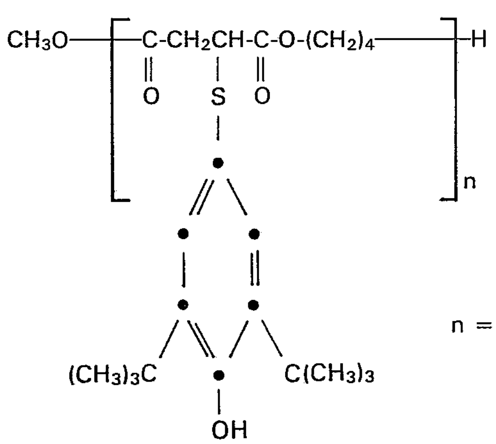

n = 15

wird zu einer weissen Festsubstanz mit Smp. 66-79°C getrocknet. Ausbeute: 13,6 g.

Anal.

| | |
|---|---|
| Berechnet für $[C_{22}H_{32}O_5S]n$: | C 64,7; H 7,9; S 7,8 |
| Gefunden: | C 64,9; H 8,1; S 7,8 |

*Beispiel 10*

*Stabilisierung von schlagfestem Polystyrol*

Man stellt eine Lösung von 8 Gew.-% Polybutadien-Kautschuk (Firestone DIENE 55® ) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat, um später die polymerisierte Probe leicht aus dem Reaktionsgefäss ablösen zu können. Die Polymerisation wird in einem Gefäss durchgeführt, das mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121°C erwärmt, und diese Temperatur wird aufrecht erhalten, bis zwischen 30 und 35% des Monomeren umgesetzt sind (Zeitdauer ca. 2,5 Stunden). Währen der Polymerisation wird heftig gerührt, und die Rührgeschwindigkeit wird so eingestellt, dass Polymerpartikel von 2 bis 4 µm Grösse entstehen. Anschlies-

send werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt:
eine Stunde bei 100°C um die Anfangstemperatur vorzugeben, eine weitere Stunde, um 140°C zu erreichen und anschliessend weitere 8 Stunden, wobei die Temperatur jede Stunde um 10°C erhöht wird, bis schliesslich ein Maximum von 220°C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200°C für 45 Minuten bei einem Druck von 1,33 mbar belassen, um flüchtige Bestandteile zu entfernen. Nach dieser Behandlung wird der Block sofort in einer heizbaren hydraulischen Presse bei 205°C zwischen zwei Aluminiumfolien zu einem dicken Stab gepresst (drei Minuten Erhitzen, fünf Minuten ungeheizt).

Der Stab wird mittels einer Bandsäge zerkleinert und anschliessend granuliert. Alle Polymermischungen werden bei 205°C extrudiert und dann zerkleinert. Die Pellets werden unter Druck bei 205°C in dehnbare 3,2 mm dicke Streifen verformt. Diese Probestreifen werden dann bei 150°C in einem Umluftofen gealtert, wobei sie auf Glasplatten, die sich wiederum auf rotierenden Trägern befinden, aufgebracht sind. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts; Ziehgeschwindigkeit: 5 mm/Minute, ASTM D-638).

In der folgenden Tabelle 1 sind die Ergebnisse der Dehnbarkeitsmessungen und der Messungen des Yellowness-Index von bei 150°C ofengealterten Proben angegeben.

TABELLE 1

Dehnbarkeitsmessungen und Yellowness Index von bei 150°C ofengealterten Proben mit Stabilisator

| Additiv (Produkt von Beispiel) | Stabilisatormenge (Gew.-%) | Dehnbarkeit der Probe (%) und Dauer der Hitzebelastung (h) | | | | |
|---|---|---|---|---|---|---|
| | | 0 h | 0,5 h | 1 h | 1,5 h | 2 h |
| 6 | 0,1 | 45 | 52 | 48 | 19 | — |
| 8 | 0,1 | 61 | 17 | — | 10 | 9 |
| — | — | 33 | 7 | 7 | 3 | 3 |

| Additiv (Produkt von Beispiel) | Stabilisatormenge (Gew.-%) | Yellowness Index und Dauer der Hitzebelastung (h) | | | | |
|---|---|---|---|---|---|---|
| | | 0 h | 0,5 h | 1 h | 1,5 h | 2 h |
| 6 | 0,1 | -2 | 4 | 8 | 10 | — |
| 8 | 0,1 | 4 | 8 | 17 | 20 | 22 |
| — | — | 7 | 18 | 30 | 38 | 43 |

*Beispiel 11*

*Stabilisierung von Polypropylen* (Lichtstabilität)

Unstabilisiertes Polypropylenpulver (Hercules Profax® 6501) wird mit 0,2 Gew.-% eines Additivs gemischt. Diese Mischung wird anschliessend bei 182°C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220°C und 12 bar zu einer 0,13 mm dicken Probe verformt. Diese Probe wird ultraviolettem Licht bis zur Zersetzung ausgesetzt. Als Zersetzungszeitpunkt wird diejenige Anzahl von Stunden gewertet, nach der die Probe die ersten Zerfallserscheinungen zeigt (braune Ecken, Risse). Die Messdaten sind in der folgenden Tabelle 2 dargestellt:

TABELLE 2

| Additiv (Produkt von Beispiel) | Zahl der Stunden bis zur Zersetzung der bestrahlten Proben |
|---|---|
| 2 | 380 |
| 4 | 410 |
| 6 | 450 |
| 8 | 360 |
| — | 200-300 |

*Beispiel 12*

*Stabilisierung von Polypropylen* (Oxidationsstabilität)

Um die Oxidationsstabilität von Polypropylen, das 0,2 Gew.-% eines erfindungsgemässen Additivs oder einer synergistisch wirkenden Formulierung bestehend aus 0,1 Gew.-% eines Additivs und 0,3 Gew.-% Distearyldithiodipropionat (DSTDP) enthält, zu untersuchen, werden Plättchen von 0,64 mm Dicke hergestellt und in einem Umluftofen bei 150°C gealtert. Als Zersetzungszeitpunkt wird diejenige Anzahl von Stunden gewertet, nach der die Probe die ersten Zerfallserscheinungen zeigt (braune Ecken, Risse).

Die Messdaten sind in Tabelle 3 dargestellt:

TABELLE 3

| Additive (Produkt von Beispiel) | Zahl der Stunden bis zur Zersetzung der Proben | |
|---|---|---|
| | 0,2% Additiv | 0,1% Additiv + 0,3% DSTDP |
| 2 | 330 | 580 |
| 4 | 410 | 570 |
| 6 | 170 | 240 |
| 8 | 130 | 320 |
| — | <20 | <20 |

**Patentansprüche**

1. Verbindung der Formel I

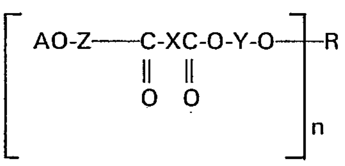

(I),

worin A $C_1$-$C_{12}$ Alkyl, $C_5$-$C_6$ Cycloalkyl, unsubstituiertes oder durch $C_1$-$C_{12}$ Alkyl substituiertes Phenyl oder eine Gruppe

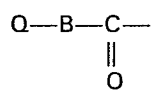

bedeutet, worin Q eine Gruppe der Formel

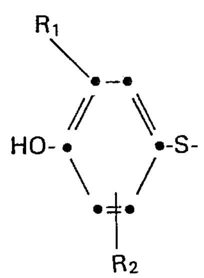

ist und B $C_2$-$C_6$ Alkylen bedeutet,

$R_1$ und $R_2$ unabhängig voneinander Wasserstoff, $C_1$-$C_{18}$ Alkyl, $C_5$-$C_6$ Cycloalkyl, Phenyl, durch $C_1$-$C_{12}$ Alkyl substituiertes Phenyl, $C_7$-$C_9$ Aralkyl oder durch $C_1$-$C_{12}$ Alkyl substituiertes $C_7$-$C_9$ Aralkyl sind,

X als gegebenenfalls wiederholt vorkommendes Symbol jeweils gleich oder verschieden $C_2$-$C_{10}$ Alkylen, durch eine Gruppe Q substituiertes $C_2$-$C_{10}$ Alkylen, $C_6$-$C_{10}$ Arylen bedeutet,

Y als gegebenenfalls wiederholt vorkommendes Symbol jeweils gleich oder verschieden ununterbrochenes oder durch ein oder zwei Sauerstoffatome unterbrochenes, unsubstituiertes oder durch eine Gruppe -O-A substituiertes $C_2$-$C_6$ Alkylen bedeutet,

Z eine direkte Bindung oder $C_2$-$C_6$ Alkylenoxy ist, wobei das Sauerstoffatom an die Carboxylgruppe gebunden und der Alkylenrest ununterbrochen oder durch ein oder zwei Sauerstoffatome unterbrochen ist,

R Wasserstoff oder eine Gruppe A bedeutet,

n eine Zahl von 1 bis 100 ist,

mit der Bedingung, dass mindestens eines von X und Y eine Gruppe Q enthält.

2. Verbindungen gemäss Anspruch 1, der Formel I, worin n die Zahl 1 bedeutet.

3. Verbindungen gemäss Anspruch 1, der Formel I, worin A $C_1$-$C_8$ Alkyl, Phenyl oder eine Gruppe der Formel

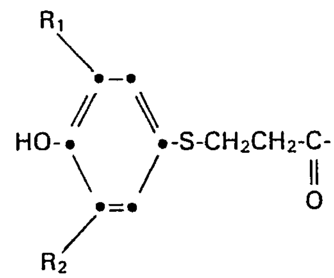

ist, $R_1$ und $R_2$ unabhängig voneinander $C_1$-$C_8$ Alkyl bedeuten,

X als gegebenenfalls wiederholt vorkommendes Symbol jeweils gleich oder verschieden $C_2$-$C_6$ Alkylen, welches durch eine Gruppe Q' der Formel

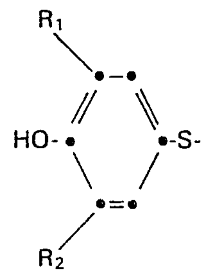

substituiert ist, worin $R_1$ und $R_2$ die in diesem Anspruch bereits angegebene Bedeutung haben, oder Phenylen bedeutet,

Y als gegebenenfalls wiederholt vorkommendes Symbol jeweils gleich oder verschiedenen unsubstituiertes oder durch eine Gruppe -O-A, worin A die in diesem Anspruch bereits angegebene Bedeutung hat, substituiertes $C_2$-$C_6$ Alkylen ist oder 3-Oxapentamethylen bedeutet,

Z eine direkte Bindung, $C_2$-$C_6$ Alkylenoxy oder 3-Oxapentamethylenoxy ist,

R Wasserstoff oder eine Gruppe A ist, wobei A die in diesem Anspruch bereits angegebene Bedeutung hat,

n die Zahlen 1 bis 50 ist,

mit der Bedingung dass,

wenn n = 1 ist, mindestens eines von X oder Y eine Gruppe Q' enthält und,

wenn n = 2-50 ist, mindestens zwei von A, X oder Y eine Gruppe Q' enthalten.

4. Verbindungen gemäss Anspruch 1, der Formel I, worin A $C_1$-$C_8$ Alkyl oder eine Gruppe der Formel

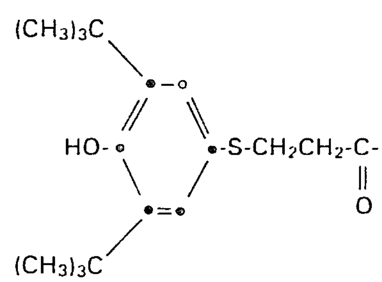

bedeutet,

X als gegebenenfalls wiederholt vorkommendes

Symbol jeweils gleich oder verschieden $C_2$-$C_6$ Alkylen, 1,2-Phenylen oder eine Gruppe der Formel

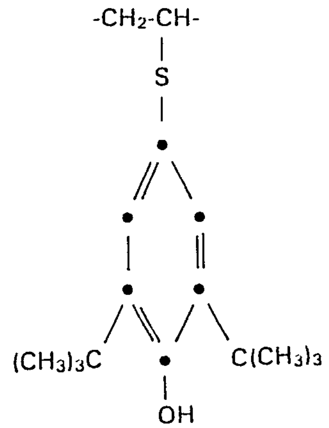

ist,

Y als gegebenenfalls wiederholt vorkommendes Symbol jeweils gleich oder verschieden $C_2$-$C_6$ Alkylen, 3-Oxapentamethylen oder eine Gruppe der Formel

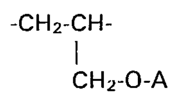

worin

A die in diesem Anspruch angegebene Bedeutung hat, bedeutet,

Z eine direkte Bindung, Ethylenoxy oder 3-Oxapentamethylenoxy ist,

R Wasserstoff oder A ist, wobei A die in diesem Anspruch bereits angegebene Bedeutung hat,

n die Zahlen 2 bis 25 bedeutet,

mit der Bedingung, dass mindestens zwei von A, X oder Y eine Gruppe der Formel

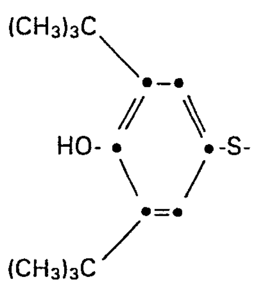

enthalten.

5. Stoffzusammensetzung enthaltend ein gegen oxidativen, thermischen oder strahlungsinduzierten Abbau empfindliches organisches Material und mindestens eine Verbindung der Formel I gemäss Anspruch 1 als Stabilisator.

6. Stoffzusammensetzung gemäss Anspruch 5, worin unter dem organischen Material ein synthetisches Polymer zu verstehen ist.

7. Stoffzusammensetzung gemäss Anspruch 6, worin unter dem synthetischen Polymer ein Polyolefin, schlagfestes Polystyrol, ABS-Harz, Polybutadien, EPM, EPDM, SBR, Nitrilkautschuk oder natürlicher Kautschuk zu verstehen ist.

8. Stoffzusammensetzung gemäss Anspruch 5, enthaltend zusätzlich mindestens einen Costabilisator ausgewählt aus der Gruppe bestehend aus Antioxidantien, Lichtschutzmittel, Metalldesaktivatoren, Phosphite, Phosphonite und Thiosynergisten.

**Claims**

1. A compound of the formula I

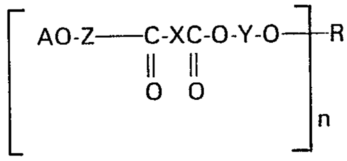 (I),

wherein

A is alkyl of 1 to 12 carbon atoms, cycloalkyl of 5 to 6 carbon atoms, phenyl, phenyl substituted by alkyl of 1 to 12 carbon atoms, or is a group of the formula

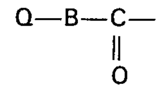

wherein Q is a group of the formula

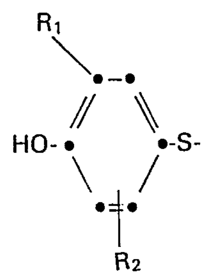

and B is alkylene of 2 to 6 carbon atoms;

$R_1$ and $R_2$ are independently hydrogen, alkyl of 1 to 18 carbon atoms, cycloalkyl of 5 to 6 carbon atoms, phenyl, phenyl substituted by alkyl of 1 to 12 carbon atoms, aralkyl of 7 to 9 carbon atoms or said aralkyl substituted by alkyl of 1 to 12 carbon atoms;

X which can be the same or different in each repeating unit is alkylene of 2 to 10 carbon atoms which is unsubstituted or substituted by a group Q or is arylene of 6 to 10 carbon atoms;

Y which can be the same or different in each repeating unit is alkylene of 2 to 6 carbon atoms, which is uninterrupted or interrupted by one or two oxygen atoms and is unsubstituted or substituted by a group -O-A;

Z is a direct bond or is alkyleneoxy of 2 to 6 carbon atoms, the oxygen atom being attached to the carbonyl group and the alkylene moiety being uninterrupted or interrupted by one or two oxygen atoms;

R is hydrogen or A;

n is 1 to 100;

with the proviso that at least one of X and Y contains a group Q.

2. A compound according to claim 1 of the formula I, wherein n is 1.

3. A compound according to claim 1 of the formula I, wherein A is alkyl of 1 to 8 carbon atoms, phenyl or a group of the formula

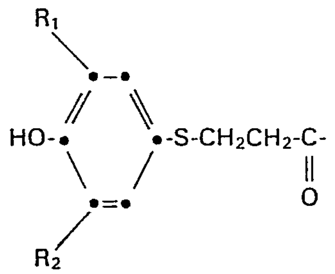

$R_1$ and $R_2$ are independently alkyl of 1 to 8 carbon atoms;

X which can be the same or different in each repeating unit is alkylene of 2 to 6 carbon atoms which is substituted by a group Q′ of the formula

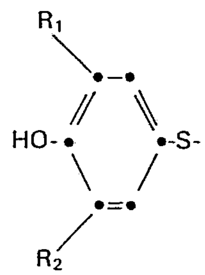

wherein $R_1$ and $R_2$ have the meanings given in this claim, or X is phenylene;

Y which can be the same or different in each repeating unit is alkylene of 2 to 6 carbon atoms which is unsubstituted or substituted by a group

-O-A

wherein A has the meanings given in this claim, or Y is 3-oxapentamethylene;

Z is a direct bond, alkyleneoxy of 2 to 6 carbon atoms or 3-oxapentamethyleneoxy;

R is hydrogen or A, A having the meanings given in this claim;

n is 1 to 50,

with the proviso that

if n is 1 at least one of X or Y contains a group Q′ and if n is 2-50, at least two of A, X or Y contain a group Q′.

4. A compound according to claim 1 of the formula I, wherein A is alkyl of 1 to 8 carbon atoms or a group of the formula

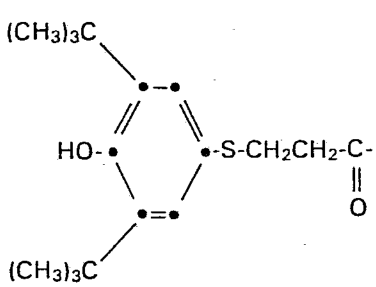

X which can be the same or different in each repeating unit is alkylene of 2 to 6 carbon atoms, 1,2-phenylene or a group of the formula

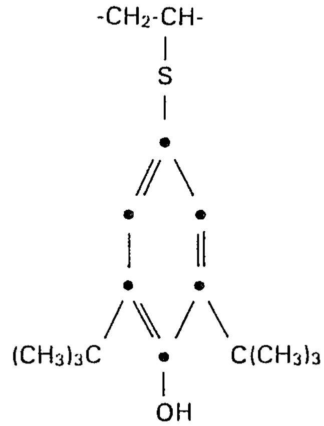

Y which can be the same or different in each repeating unit is alkylene of 2 to 6 carbon atoms, 3-oxapentamethylene or a group of the formula

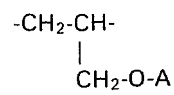

wherein A has the meaning given in this claim;

Z is a direct bond, ethyleneoxy or 3-oxapentamethyleneoxy;

R is hydrogen or A, A having the meaning given in this claim;

n is 2 to 25;

with the proviso that at least two of A, X or Y contain a group of the formula

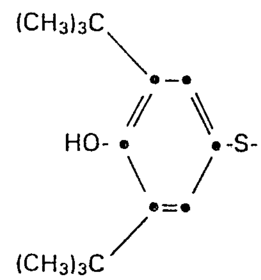

5. A composition comprising an organic material subject to oxidative, thermal or radiation-induced degradation and at least one compound of formula I according to claim 1 as stabiliser.

6. A composition according to claim 5, wherein the organic material is a synthetic polymer.

7. A composition according to claim 6, wherein said synthetic polymer is selected from the group consisting of polyolefins, impact polystyrene, acrylonitrile/butadiene/styrene resin, polybutadiene, EMP, EPDM, styrene/butadiene rubber, nitrile rubber or natural rubber.

8. A composition according to claim 5 containing additionally at least one costabilizer selected from the group consisting of antioxidants, light stabilizers, metal deactivators, phosphites, phosphonites and thiosynergists.

**Revendications**

1. Composés répondant à la formule I:

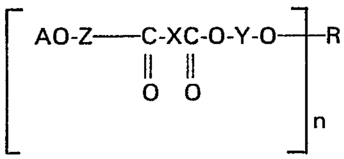

(I)

dans laquelle:

A représente un alkyle en $C_1$-$C_{12}$, un cycloalkyle en $C_5$ ou $C_6$, un phényle non substitué ou porteur d'un alkyle en $C_1$-$C_{12}$ ou un radical répondant à la formule:

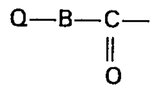

dans laquelle:

Q représente un radical de formule:

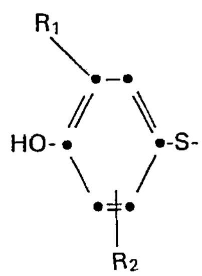

où $R_1$ et $R_2$ représentent chacun, indépendamment l'un de l'autre, l'hydrogène, un alkyle en $C_1$-$C_{18}$, un cycloalkyle en $C_5$ ou $C_6$, un phényle, un phényle porteur d'un alkyle en $C_1$-$C_{12}$, un aralkyle en $C_7$-$C_9$ ou un aralkyle en $C_7$-$C_9$ porteur d'un alkyle en $C_1$-$C_{12}$, et

B représente un alkylène en $C_2$-$C_6$,

X ou, lorsqu'il y en a plusieurs, chacun des X représente, indépendamment du ou des autres, un alkylène en $C_2$-$C_{10}$, un alkylène en $C_2$-$C_{10}$ porteur d'un radical Q ou un arylène en $C_6$-$C_{10}$,

Y ou, lorsqu'il y en a plusieurs, chacun des Y représente, indépendamment du ou des autres, un radical alkylène en $C_2$-$C_6$ non interrompu ou interrompu par un ou deux atomes d'oxygène, non substitué ou porteur d'un radical -O-A,

Z représente une liaison directe ou un alkylène-oxy en $C_2$-$C_6$ dont l'atome d'oxygène est uni au radical carbonyle et dont la partie alkylène n'est pas interrompue ou est interrompue par un ou deux atomes d'oxygène,

R représente l'hydrogène ou un radical A et

n représente un nombre de 1 à 100,

et dans laquelle au moins l'un des radicaux X et Y contient un radical Q.

2. Composés de formule I selon la revendication 1 dans lesquels n désigne le nombre 1.

3. Composés de formule I selon la revendication 1 dans lesquels:

A représente un alkyle en $C_1$-$C_8$, un phényle ou un radical répondant à la formule:

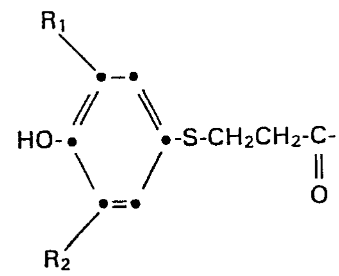

dans laquelle $R_1$ et $R_2$ représentent chacun, indépendamment l'un de l'autre, un alkyle en $C_1$-$C_8$,

X représente, ou les X représentent chacun indépendamment les uns des autres, un alkylène en $C_2$-$C_6$ qui peut porter un radical Q' de formule:

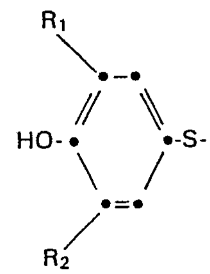

où $R_1$ et $R_2$ ont les signification qui viennent d'être données dans cette revendications, ou un phénylène,

Y représente, ou les Y représentent chacun, indépendamment les uns des autres, un alkylène en $C_2$-$C_6$ non substitué ou porteur d'un radical -O-A dont le symbole A a la signification qui vient d'être donnée dans cette revendication, ou un oxa-3 pentaméthylène,

Z représente une liaison directe, un alkylène-oxy en $C_2$-$C_6$ ou un oxa-3 pentaméthylène-oxy,

R représente l'hydrogène ou un radicale A, ce radical A ayant la signification qui vient d'être donnée dans cette revendication, et

n designe un nombre de 1 à 50,

avec la condition que:

- dans le cas où n est égal à 1, au moins l'un des radicaux X et Y contienne un radical Q', et que
- dans le cas où n désigne un nombre de 2 à 50, au moins deux des radicaux A, X et Y contiennent un radical Q'.

4. Composés de formule I selon la revendication 1 dans lesquels:

A représente un alkyle en $C_1$-$C_8$ ou un radical de formule:

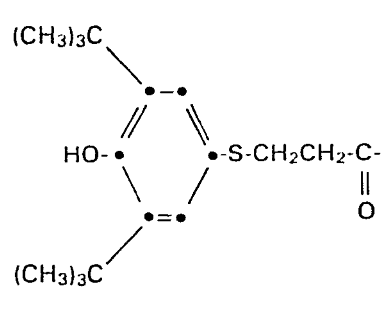

X représente, ou les X représentent chacun, indépendamment les uns des autres, un alkylène en $C_2$-$C_6$, un phénylène-1,2 ou un radical de formule:

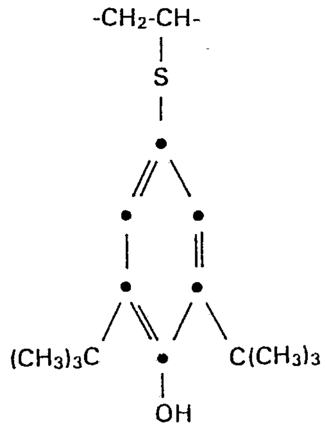

Y représente, ou les Y représentent chacun, indépendamment les uns des autres, un alkylène en $C_2$-$C_6$, un oxa-3 pentaméthylène ou un radical répondant à la formule:

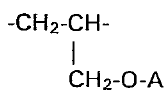

dans laquelle:

A a la signification qui vient d'être donnée dans cette revendication,

Z représente une liaison directe ou un radical éthylène-oxy ou oxa-3 pentaméthylène-oxy,

R représente l'hydrogène ou un radical A, ce symbole A ayant la signification qui vient d'être donnée dans cette revendication, et

n désigne un nombre de 2 à 25,

avec la condition qu'au moins deux des radicaux A, X et Y contiennent un radical de formule:

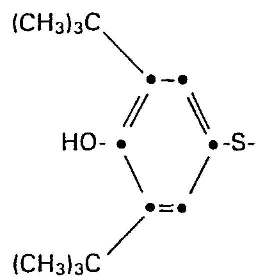

5. Composition contenant une matière organique sensible à la dégradation due à l'oxydation, à la chaleur ou au rayonnement et, comme stabilisant, au moins un composé de formule I selon la revendication 1.

6. Composition selon la revendication 5 dans laquelle la matière organique est un polymère synthétique.

7. Composition selon la revendication 6 dans laquelle le polymère synthétique est une polyoléfine, un polystyrène haute résilience, une résine ABS, le polybutadiène, l'EPM, l'EPDM, le SBR, le caoutchouc nitrile ou le caoutchouc naturel.

8. Composition selon la revendication 5 qui contient en outre au moins un co-stabilisant pris dans l'ensemble constitué par les anti-oxydants, les stabilisants à la lumière, les désactivants de métaux, les phosphites, les phosphonites et les agents de synergie sulfurés.